# EUROPÄISCHE PATENTSCHRIFT

(11) **EP 0 316 816 B1**
(45) Veröffentlichungstag und Bekanntmachung des Hinweises auf die Patenterteilung: **16.06.1993**
(21) Anmeldenummer: 88118873.4
(22) Anmeldetag: 11.11.1988
(51) Int. Cl.: A61B 17/28

(54) **Chirurgische Fasszange**
Surgical forceps
Pince chirurgicale

(30) Priorität: 13.11.1987 DE 3738692
(43) Veröffentlichungstag der Anmeldung: 24.05.1989
(73) Patentinhaber: Haberl, Hannes, Dr., D-81667 München (DE)
(72) Erfinder: Haberl, Hannes, Dr., D-81667 München (DE)
(74) Vertreter: Pohlmann, Eckart, Dipl.-Phys.

(56) Entgegenhaltungen:
- EP-A- 0 069 942
- EP-A- 0 212 308
- DE-A- 2 945 407
- DE-A- 3 035 416
- DE-A- 3 447 769
- DE-A- 3 500 444
- DE-B- 1 225 813
- DE-C- 685 047
- DE-U- 8 715 139
- FR-A- 1 430 073
- FR-A- 2 469 912
- FR-A- 2 535 196
- US-A- 4 644 951

## Beschreibung

Die Erfindung betrifft eine chirurgische Fasszange mit einem Zangengriff, der über zwei ineinander angeordnete und axial relativ zueinander bewegbare Rohrelemente mit einem Zangenmaul derart verbunden ist, daß das Zangenmaul über eine Betätigung des Zangengriffes geöffnet und geschlossen werden kann, einer Endoskopeinrichtung mit einem Endoskopstab, der im inneren Rohrelement angeordnet ist, und einer Absaugeinrichtung, die am Zangenmaul mündet.

Eine derartige Fasszange, die aus der US-46 07 620 bekannt ist, wird bei operativen Eingriffen zur Gewebeentnahme benutzt.

Aus der EP-A-02 12 308 ist eine weitere chirurgische Fasszange mit einem Zangengriff bekannt, der über ein Rohrelement und eine in diesem Rohrelement exzentrisch angeordnete massive Betätigungsstange mit einem Zangenmaul so verbunden ist, daß das Zangenmaul über eine Betätigung des Zangengriffes geöffnet und geschlossen werden kann, wobei weiterhin eine Absaugeinrichtung vorgesehen ist, die am Zangenmaul mündet.

Aus der DE-A-30 35 416 ist es weiterhin bekannt, bei einer Einrichtung zur Gewebeprobenentnahme zur optischen Überwachung der Gewebeprobeentnahme eine Lichtquelle am Gewebeprobenentnahmeteil vorzusehen, wo die Gewebeproben genommen und angesaugt werden, so daß dieser Vorgang direkt optisch kontrolliert werden kann. Dazu ist ein entsprechender Lichtleiter vorgesehen.

Die Fasszange der eingangs genannten Art, die aus der US-46 07 620 bekannt ist, stellt ein Kombinationsgerät dar, bei dem das Endoskop, die eigentliche Fasszange und die Absaugeinrichtung miteinander kombiniert sind. Aufgrund der normalerweise sehr engen Körperkanäle, über die eine derartige Fasszange eingeführt werden kann, sind dem Gesamtdurchmesser der Fasszange enge Grenzen gesetzt, so daß es bisher nur möglich war, eine derartige Fasszange für die Biopsie, jedoch nicht für Operationen einzusetzen, bei denen größere Mengen an Gewebe entfernt und abgesaugt werden müssen. Das gilt beispielsweise für Bandscheibenoperationen, bei denen u.U. die gesamte Bandscheibe ausgeräumt werden muß.

Die der Erfindung zugrunde gelegte Aufgabe besteht daher darin, eine Fasszange nach dem Gattungsbegriff des Patentanspruchs 1 so weiterzubilden, daß sie auch bei Operationen, bei denen größere Gewebemengen entfernt werden müssen, beispielsweise bei Bandscheibenoperationen einsetzbar ist, bei denen die gesamte Bandscheibe ausgeräumt werden muß.

Diese Aufgabe wird gemäß der Erfindung dadurch gelöst, daß das äußere Rohrelement ausgehend vom Zangengrriff über einen Teil in Längsrichtung als Zwillingsrohr ausgebildet ist, in dessen einem Rohrteil das innere Rohrelement aufgenommen ist, während der andere Rohrteil das Absaugrohr der Absaugeinrichtung bildet und mit einer Saugeinrichtung verbindbar ist, wobei die Rohrteile des Zwillingsrohres an einer Stelle im Abstand vom zangenmaulseitigen Ende ineinander und in das innere Rohrelement münden und der Endoskopstab so angeordnet ist, daß er bei Schließen des Zangenmaules wenigstens über die Mündung der Rohrteile des Zwillingsrohres zurückgezogen und beim Öffnen des Zangenmaules wieder bis zum Grund des Zangenmaules vorgeschoben wird.

Aufgrund der erfindungsgemäßen Ausbildung des äußeren Rohres teilweise in Längsrichtung als Zwillingsrohr und die verschiebbare Ausbildung des Endoskopstabes derart, daß dieser je nach Lage den Ansaugkanal freigibt oder schließt, wird es möglich, den vorderen Abschnitt der Fasszange trotz der Ausbildung des gesamten Gerätes als Kombinationsgerät mit Endoskop und Absaugeinrichtung so schmal auszubilden, daß ein Einführen in enge Körperkanäle möglich ist, über die beispielsweise bei Bandscheibenoperationen der Operationsbereich zugänglich ist.

Bevorzugte Weiterbildungen der erfindungsgemäßen Faßzange sind Gegenstand der Patentansprüche 2 und 3.

Im folgenden wird anhand der zugehörigen Zeichnung ein besonders bevorzugtes Ausführungsbeispiel der Erfindung näher beschrieben. Es zeigen
- Fig. 1: eine schematische Seitenansicht einer Faßzange,
- Fig. 2: eine perspektivische Ansicht des Endabschnittes der Schaftelemente mit dem daran ausgebildeten Zangenmaul, und
- Fig. 3: eine teilweise geschnittene Seitenansicht eines Ausführungsbeispiels der Erfindung.

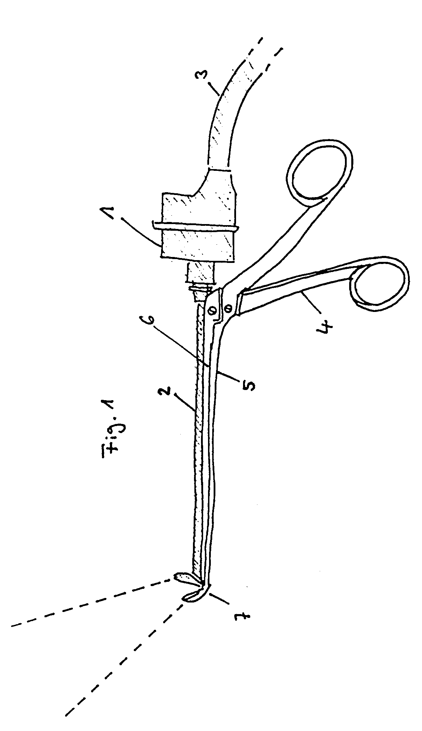

Die in Fig. 1 dargestellte Faßzange umfaßt in üblicher Weise einen Zangengriff 4 und Schaftelemente 5, 6, die den Zangengriff 4 mit einem Zangenmaul 7 so verbinden, daß das Zangenmaul 7 durch eine Betätigung des Zangengriffs 4 geöffnet und geschlossen werden kann. Die Betätigung des Zangengriffes 4 führt zu einer Relativverschiebung der Schaftelemente 5 und 6, die auf den beweglichen Teil 8 des Zangenmaules 7 so übertragen wird, daß dieser auf und zu klappt.

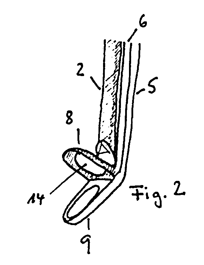

Eine Endoskopeinrichtung 1 mit einem Endoskopstab 2 ist an dem beweglichen Schaftteil 6, beispielsweise durch Kleben oder in anderer Weise, so angebracht, daß der Endoskopstab 2 am Zangenmaul 7 mündet. Wie es in Fig. 2 dargestellt ist, ist in dem beweglichen Teil 8 des Zangenmaules 7, der der Mündung des Endoskopstabes 2 gegenüberliegt, eine Sichtöffnung 14 ausgebildet, durch die das Endoskop mit seinem Endoskopstab hindurch sehen kann.

Das Endoskop 1 ist über ein Verbindungskabel 3 mit einem üblichen Monitor verbunden.

Bei der Arbeit mit der in Fig. 1 und 2 dargestellten Faßzange wird die Faßzange mit dem Zangenmaul 7 und den Schaftteilen 5, 6, sowie dem daran angebrachten Endoskopstab in den Körper eingeführt, die gewünschte Gewebeentnahme erfolgt bei gleichzeitiger visueller Überwachung des Operationsbereiches im Inneren des Körpers am Bildschirm des Monitors. Dabei ist die Endfläche des Endoskopstabes 2 unter einem Winkel zur Längsachse geschnitten, der dem Winkel des Zangenmaules entspricht, das zur Längsachse abgewinkelt angeordnet sein kann, so daß sich der gewünschte Blickwinkel und das gewünschte Gesichtsfeld ergeben, wie es in gestrichelten Linien in Fig. 1 dargestellt ist.

Nach Einführen der Faßzange wird durch Betätigen des Zangengriffes 4 das gewünschte Gewebe mit dem Zangenmaul 7 erfaßt, was am Monitor überwacht werden kann, wonach die Faßzange mit geschlossenem Zangenmaul und dem darin gehaltenen Gewebe herausgezogen wird.

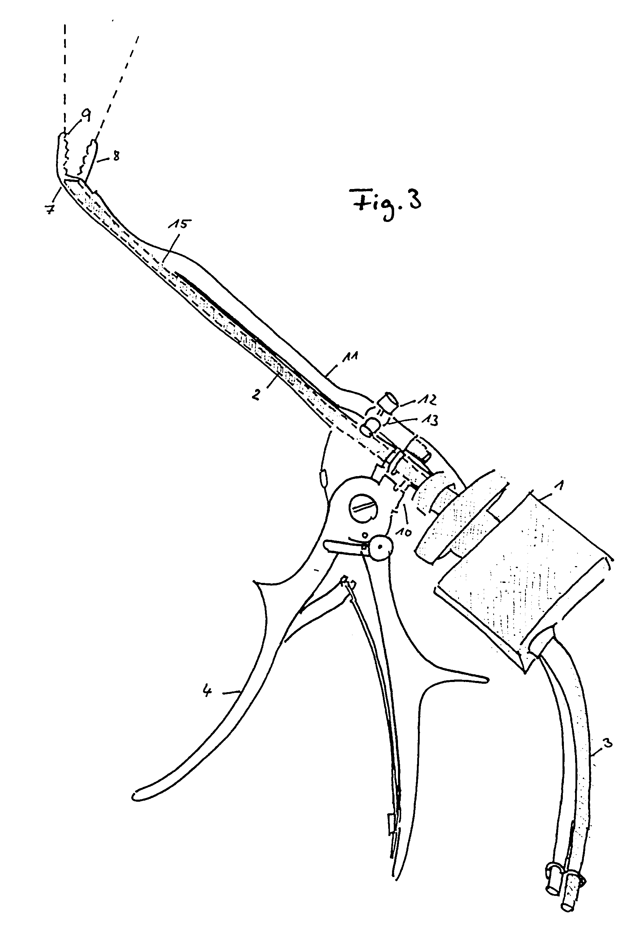

Bei dem in Fig. 3 dargestellten Ausführungsbeispiel der Erfindung ist es möglich, fortlaufend Gewebe zu entnehmen, ohne daß die Faßzange aus dem Körper herausgezogen werden muß.

Das in Fig. 3 dargestellte Ausführungsbeispiel der erfindungsgemäßen Faßzange umfaßt wiederum in üblicher Weise einen Zangengriff 4, der über Schaftelemente mit einem Zangenmaul 7 verbunden ist, die gegeneinander bewegbar sind, um das Zangenmaul 7 zu öffnen und zu schließen. Die Schaftelemente sind in Form von zwei ineinander angeordneten konzentrischen Rohren ausgebildet, die axial gegeneinander über einen Mechanismus 10 am Zangengriff 4 bewegbar sind, wenn der Zangengriff 4 betätigt wird. Durch das Verschieben des einen Rohres im anderen Rohr wird der bewegliche Teil 8 des Zangenmauls 7 gegenüber dem ortsfesten Teil 9 auf und zu geklappt.

Bei dem in Fig. 3 dargestellten Ausführungsbeispiel ist weiterhin eine Absaugeinrichtung vorgesehen, die dadurch ausgebildet ist, daß der Schaftteil der Faßzange teilweise als Zwillings- oder Doppelrohr ausgebildet ist. Das erfolgt dadurch, daß ein zweites Rohr an das äußere Rohr der beiden ineinander beweglichen rohrförmigen Schaftelemente angesetzt ist, wobei dieses zusätzliche Rohr an einer Stelle im Abstand vom zangenmaulseitigen Ende in das innere und äußere Rohr der beiden zueinander beweglichen Verbindungsrohre mündet und mit einer Absaugeinrichtung verbunden ist. Im Inneren des inneren Verbindungsrohres ist der Endoskopstab 2 eines Endoskops angeordnet. Der Endoskopstab 2 ist gleichfalls mit dem Öffnungs- und Schließmechanismus 10 des Zangengriffes 4 so verbunden, daß der Endoskopstab 2 beim Schließen des Zangenmaules 7 bis zur Mündungsstelle 15 zwischen dem Absaugrohrteil und dem Betätigungsrohrteil des Zwillingsrohres 11 zurückgezogen wird und beim Öffnen des Zangenmaules 7 wieder bis zum Boden des Zangenmaules 7 vorgeschoben wird.

Vorzugsweise ist in dem Rohrteil des Zwillingsrohres 11, der an die Absaugeinrichtung angeschlossen ist, eine Ventilklappe 13 oder ein ähnlich gestaltetes Ventil vorgesehen, das diesen Rohrteil schließen kann. Ein Anschlußstutzen 12 zum Anschließen an eine Spülflüssigkeitsquelle ist außen an diesem Teil des Zwillingsrohres 11 vorgesehen und kann gleichfalls durch die Ventilklappe 13 geschlossen werden. Diese Ausbildung dient dazu, das Absaugrohr zu spülen, was dadurch erfolgt, daß das Rohr an dem mit der Absaugeinrichtung verbundenen Ende geschlossen und der Anschlußstutzen 12 geöffnet wird. Während des normalen Betriebes ist der Anschlußstutzen 12 geschlossen und ist die Stellung des Ventiles 13 so, daß der Absaugrohrteil des Zwillingsrohres 11 zur Absaugeinrichtung hin offen ist.

Beim Einsatz des in Fig. 3 dargestellten Ausführungsbeispiels der Erfindung wird wiederum die Faßzange mit ihrem Schaftteil und dem darin angeordneten Endoskopstab 2 in den Körper eingeführt, in dem der Eingriff vorzunehmen ist. Bei geöffnetem Zangenmaul 7 können die über den Endoskopstab 2, der am Grund des Zangenmaules 7 mündet, übertragenen Bilder am Monitor betrachtet werden. Wenn unter dieser optischen Überwachung das Zangenmaul 7 geschlossen wird, was über eine Betätigung des Griffes 4 erfolgt, wird gleichzeitig über den Öffnungs- und Schließmechanismus 10 der Endoskopstab um eine bestimmte Strecke bis zur Mündung 15 der Rohre des Zwillingsrohres 11 zurückgezogen, so daß der am Absaugrohr des Zwillingsrohres 11 liegende Saugdruck an dem distal der Mündungsstelle liegendem Rohrabschnitt einschließlich des Zangenmaules liegt und das davon erfaßte Gewebe abgesaugt werden kann. Anschließend wird das Zangenmaul wieder geöffnet, wobei der Öffnungs-und Schließmechanismus den Endoskopstab 2 wieder bis zum Grund des Zangenmaules vorschiebt, wodurch die Mündung des Absaugrohres an der Mündungsstelle 15 verschlossen wird. Der daran liegende Unterdruck erreicht daher nicht das Zangenmaul. Wiederum kann die gewünschte Stelle im Körperinneren betrachtet und aufgesucht werden und kann die gewünschte Gewebeentnahme über den Monitor kontrolliert erfolgen. Die Hin- und Herbewegungen des Endoskopstabes 2 haben die weitere Wirkung, daß der vordere Endabschnitt des Schaftteiles mechanisch gereinigt wird und somit ein Anhaften oder gar Verstopfen durch Gewebeteile verhindert wird.

Da bei dem in Fig. 3 dargestellten Ausführungsbeispiel eine Absaugeinrichtung vorgesehen ist, ist es nicht mehr notwendig, bei jeder Gewebeentnahme, d.h. bei jedem Öffnen und Schließen des Zangenmaules, die gesamte Faßzange aus dem Körper herauszuziehen. Die Zange kann im Körper verbleiben, die Gewebeentnahme erfolgt durch abwechselndes Öffnen und Schließen des Zangenmaules 7 mit dazwischen liegenden Absaugvorgängen. Da bei dem in Fig. 3 dargestellten Ausführungsbeispiel der Erfindung der Endoskopstab die Mündung des Absaugrohres schließt, wenn das Zangenmaul 7 geöffnet ist, ist es auch nicht notwendig, die Absaugeinrichtung an und ab zu schalten, vielmehr kann der Absaugunterdruck permanent anliegen, was eine weitere Vereinfachung bedeutet.

Der Öffnungs- und Schließmechanismus 10 am Zangengriff 4, der neben dem Öffnen und Schließen des Zangenmaules 7 gleichzeitig eine Vor- und Zurückbewegung des Endoskopstabes 2 bewirkt, kann über geeignete Übersetzungseinrichtungen so ausgebildet sein, daß die Bewegung des Zangengriffes in eine Längsbewegung des Endoskopstabes in einem bestimmten Verhältnis von beispielsweise 1:5 zu 2:5 übersetzt wird. Die Fasszangenlänge beträgt üblicherweise 15 bis 20 cm und der Endoskopstab ist am vorderen Ende wiederum so abgeschrägt, wie es dem gewünschten Blickwinkel und der vorgesehenen Schrägstellung des Zangenmaules entspricht.

## Patentansprüche

1. Chirurgische Fasszange mit
- einem Zangengriff (4), der über zwei ineinander angeordnete und axial relativ zueinander bewegbare Rohrelemente (5,6) mit einem Zangenmaul (7) derart verbunden ist, daß das Zangenmaul (7) über eine Betätigung des Zangengriffes (4) geöffnet und geschlossen werden kann,
- einer Endoskopeinrichtung mit einem Endoskopstab (2), der im inneren Rohrelement angeordnet ist, und
- einer Absaugeinrichtung, die am Zangenmaul (7) mündet, dadurch **gekennzeichnet**, daß das äußere Rohrelement ausgehend vom Zangengriff (4) über einen Teil in Längsrichtung als Zwillingsrohr (11) ausgebildet ist, in dessen einem Rohrteil das innere Rohrelement aufgenommen ist, während der andere Rohrteil das Absaugrohr der Absaugeinrichtung bildet und mit einer Saugeinrichtung verbindbar ist, wobei die Rohrteile des Zwillingsrohres (11) an einer Stelle im Abstand vom zangenmaulseitigen Ende ineinander und in das innere Rohrelement münden und der Endoskopstab (2) so angeordnet ist, daß er bei Schließen des Zangenmaules (7) wenigstens über die Mündung (15) der Rohrteile des Zwillingsrohres (11) zurückgezogen und beim Öffnen des Zangenmaules wieder bis zum Grund des Zangenmaules (7) vorgeschoben wird.

2. Chirurgische Fasszangen nach Anspruch 1 dadurch **gekennzeichnet**, daß der Endoskopstab (2) mit dem Öffnungs- und Schließmechanismus (10) des Zangengriffes (4) verbunden ist und durch diesen zusammen mit der Öffnung und Schließung des Zangenmaules (7) vorgeschoben und zurückgezogen wird.

3. Fasszangen nach Anspruch 1 oder 2 dadurch **gekennzeichnet**, daß am Absaugrohr der Absaugeinrichtung an einer Stelle nahe am Zangengriff (4) eine Ventilklappe (13) vorgesehen ist, die zwischen einer das Absaugrohr schließenden und einen außen am Absaugrohr angebrachten Stutzen (12) für einen Spülmittelanschluß öffnenden Lage und einer Lage bewegbar ist, in der sie den Stutzen (12) für einen Spülmittelanschluß schließt und das Ansaugrohr öffnet.

## Claims

1. Surgical forceps comprising
- a forceps grip (4) being connected to a forceps mouth (7) by means of two tube elements (5,6) arranged into one another and axially movably relative to each other such that the forceps mouth (7) can be opened and closed by operating the forceps grip (4)
- an endoscope means having an endoscope rod (2) located in the inner tube element and
- a suction means opening at the forceps mouth (7) characterized in that the outer tube element starting from the forceps grip and along a portion in longitudinal direction is formed as a twin tube (11) in one of its tube parts the inner tube element is contained, whereas the other tube part forms the suction tube of the suction means and is connected to an exhaust means wherein the tube parts of the twin tube (11) open into each other and into the inner tube element at a point spaced upon the end at the forceps mouth and the endoscope rod (2) is arranged such that it is withdrawn at least beyond the opening (15) of the tube parts of the twin tube (11) when the forceps mouth (7) is closed and is again advanced up to the bottom of the forceps mouth when the forceps mouth (7) is opened.

2. Surgical forceps according to claim 1 characterized in that the endoscope rod (2) is connected to the opening and closing mechanism (10) of the forceps grip (4) and is advanced and withdrawn thereby along with opening and closing the forceps mouth (7).

3. Forceps according to claim 1 or 2 characterized in that a valve flap (13) is provided at the suction tube of the suction means at a location near the forceps grip (4) which flap is movable between a position in which the suction tube is closed and a connection piece (12) for a washing agent is opened and a position in which the connection piece (12) for the washing agent is closed and the suction tube is opened.

## Revendications

1. Pince chirurgicale comprenant
- une poignée de pince (4) qui est reliée, par l'intermédiaire de deux éléments tubulaires (5, 6) disposés l'un à l'intérieur de l'autre et mobiles axialement l'un par rapport à l'autre, à une mâchoire de pince (7), de telle manière que la mâchoire (7) de pince puisse être ouverte ou fermée par la manoeuvre de la poignée (4) de la pince,
- un dispositif endoscopique comprenant une tige d'endoscope (2) qui est disposée dans l'élément tubulaire intérieur, et
- un dispositif aspirateur qui débouche au droit de la mâchoire (7) de la pince, caractérisé en ce qu'en partant de la poignée (4) de la pince, l'élément tubulaire extérieur forme, sur une partie de cet élément, dans la direction de la longueur, un tube jumeau (11) dans lequel se loge une partie tubulaire de l'élément tubulaire intérieur, tandis que l'autre élément tubulaire forme le tube suceur du dispositif aspirateur et peut être relié à un générateur d'aspiration, les parties tubulaires du tube jumeau (11) débouchant l'un dans l'autre et dans l'élément tubulaire intérieur dans une zone située à distance de l'extrémité côté mâchoire de la pince, et la tige (2) de l'endoscope est disposée de manière à être retirée au moins au-delà du débouché (15) des parties tubulaires du tube jumeau (11) lors de la fermeture de la mâchoire (7) de la pince, et à être de nouveau poussée en avant jusqu'à la base de la mâchoire (7) de la pince lors de l'ouverture de la mâchoire de la pince.

2. Pince chirurgicale selon la revendication 1, caractérisée en ce que la tige (2) de l'endoscope est reliée au mécanisme (10) d'ouverture et de fermeture de la poignée (4) de la pince et cet poussée en avant et retirée en arrière par ce mécanisme, simultanément avec l'ouverture et la fermeture de la mâchoire (7) de la pince.

3. Pince selon la revendication 1 ou 2, caractérise en ce que, sur le tube suceur du dispositif aspirateur, est prévu, dans une zone proche de la poignée (4) de la pince, un clapet de soupape (13) qui peut se déplacer entre une position qui ferme le tube suceur et ouvre une tubulure (12) de raccordement, d'un milieu de balayage, prévue extérieurement sur le tube suceur, et une position dans laquelle il ferme la tubulure (12) de raccordement de milieu de balayage et ouvre le tube suceur.
